# EUROPEAN PATENT APPLICATION

(11) **EP 4 435 430 A1**
(43) Date of publication of application: **25.09.2024**
(21) Application number: 22711856.9
(22) Date of filing: 28.01.2022
(51) Int. Cl.: G01N 33/569, G01N 33/558, G01N 33/577, G01N 33/58

(54) **KIT FOR DETECTING SARS-COV-2 ANTIGEN AND DETECTION METHOD**

(30) Priority: 16.11.2021 CN 202111357502
(71) Applicant: Daan Gene Co., Ltd., Guangzhou, Guangdong 510665 (CN)
(72) Inventor: JIANG, Xiwen, Guangdong 510665 (CN); QI, Wenchuang, Guangdong 510665 (CN); LIU, Shuang, Guangdong 510665 (CN); PAN, Xiuhua, Guangdong 510665 (CN); SU, Songkang, Guangdong 510665 (CN); XU, Hong, Guangdong 510665 (CN)
(74) Representative: Handsome I.P. Ltd
(86) International application number: PCT/CN2022/074466
(87) International publication number: WO 2023/087550

(57) **Abstract**

The present disclosure provides a kit for testing a COVID-19 antigen. The kit includes a test card, a conjugate pad and a reaction pad being arranged on the test card; wherein the conjugate pad is provided with a labeled antibody Ab1 and a labeled antibody Ab3 of the COVID-19 antigen, and a quality control line and a test line are arranged in parallel with an interval on the reaction pad, the test line being provided with a coating antibody Ab2, and the quality control line being provided with a coating antibody Ab4. With the kit, by the AIE immunofluorescence chromatographic technique, the AIE fluorescent microspheres are used as labels, and the test result of the test card is directly determined and read by using a fluorescent flashlight, or the test result is read by using a fluorescent tester. Such kits have the advantages of high sensitivity, strong specificity, simple operation, and the like.

## Description

This application is based upon and claims priority to Chinese Patent Application No. 202111357502.6, filed before China National Intellectual Property Administration on November 16, 2021 and entitled "KIT FOR TESTING COVID-19 ANTIGEN AND METHOD FOR TESTING SAME," the entire contents of which are incorporated herein by reference.

### TECHNICAL FIELD

The present disclosure relates to the technical field of COVID-19 antigen testing, and in particular, relate to a kit for testing a COVID-19 antigen and use thereof.

### BACKGROUND

Coronavirus disease 2019 is abbreviated as COVID-19. Clinical manifestations in patients with pneumonia infected with the COVID-19 include: fever, asthenia, and dry cough as the main manifestations, and nasal obstruction, runny nose and other upper respiratory symptoms as rare manifestations; and the patients may be subject to hypoxia. About half of the patients develop respiratory distress after more than a week, and severe cases progress rapidly to acute respiratory distress syndrome, septic shock, uncorrectable metabolic acidosis, and bleeding and coagulation disorders. It is worth noting that patients with severe and critical illnesses may have moderate to low fever or even no significant fever during the course of the disease. Some patients have a mild onset of symptoms and may have no fever, and most recover after 1 week. Most patients have a good prognosis, while a few are critically ill or even die.

In the face of a massive epidemic of COVID-19 infections, a large number of people need to be screened, diagnosed and treated accordingly, and the clinical demand for diagnostic reagents is extremely urgent. In addition to medical supplies such as masks and protective clothing, there was a huge shortage of testing reagents in the early days of the Wuhan outbreak and now in Italy and Spain.

For the detection of COVID-19 infections, several COVID-19 test methods have been studied, which can be mainly divided into pathogen detection, antibody detection and immunoassay according to the principle of detection and different methods. The details are as follows:
1. For the detection of pathogens, for example, the PCR (polymerase chain reaction) method is the first method applied. The preparation of virus nucleic acid specific primer sequences is relatively fast, and the theoretical specificity and sensitivity of nucleic acid detection are high. Therefore, the virus nucleic acid detection kit based on PCR method has become the earliest kit developed for the COVID-19 detection. In the Chinese "Coronavirus Pneumonia Treatment Scheme (Trial Version 7)," positive real-time fluorescent RT-PCR is used as one of the confirmatory evidences of COVID-19 infections.

However, the inventors have found that in the actual diagnosis process, the nucleic acid method revealed many problems: first, false negative problem; second, high device or testing platform requirements; third, long testing time. First, a false negative means a missed test, which can not only lead to a rapid diagnosis of a suspected patient in the clinic, but can also make the missed test a potential source of virus transmission. Second, it has been reported that even patients who have been discharged from the hospital after multiple negative nucleic acid tests have relapsed again, which is also likely to be a misdiagnosis caused by false negatives. After sampling, nucleic acid detection also includes two steps of nucleic acid extraction and PCR amplification, and the kits and equipment used include nucleic acid extraction kits, fluorescent probes, and fluorescent RT-PCR instruments. High-sensitivity RT-PCR instruments are expensive. In addition, nucleic acid testing requires a high level of laboratory cleanliness in order to avoid the effects of nucleases that may be carried by various microorganisms in the air. Finally, nucleic acid testing is a sophisticated experiment and requires a high level of operator training. As such, in the event of a large-scale outbreak, major hospital laboratories qualified for nucleic acid testing are quickly saturated, and large numbers of samples pile up without timely access to testing. It usually takes 4-6 h to complete an RT-PCR assay; however, considering the sample transportation and large sample backlog, results are usually reported in as little as 24 h.

2. Antibody assays are based on the human immune response to foreign pathogens. When infected with a pathogen, IgM antibodies specific for that immunogen usually appear in the serum within 7-10 days, and they are the "vanguard" of the body's fight against the infection. If pathogen-specific IgM is detected in the serum, it indicates that the infection has recently occurred and can be used for early diagnosis of infection. Specific IgG against the pathogen can usually be detected after 20 days. However, in the actual diagnostic process, the antibody assay reveals various problems and cannot be used as a diagnostic indicator alone for screening, but only as an auxiliary diagnostic method when the nucleic acid test is negative. In the FDA's SARS-COV-2 testing guidance, it is clearly stated that antibody testing cannot be used alone to diagnose COVID-19 and cannot rule out infection or indicate infection status. Antibody tests may also be positive for previous or current infection with other viruses such as HKU1, NL63, OC43 or 229E. Most antibody detection kits use capture ELISA to detect specific antibodies. That is, the IgM or IgG in the sample is captured by antibodies against IgM/IgG. In the case of pathogen-specific IgM or IgG, it will bind to the labeled antigen and thus become visible in the detection line of the test strip or in the corresponding well of the 96-well plate. Since the capture antibody (anti-IgM/IgG antibody) binds any IgM or IgG in the serum, the specificity of the method is based only on the specific recognition and binding of the antigen-antibody pair (i.e., the antibody in the serum recognizes the viral protein provided by the kit). This results in false positives due to non-specific binding in complex sample compositions.

3. The immunocolloidal gold (ICA) method for the detection of COVID-19 antigens uses high electron-density colloidal gold as a marker on antibodies or antigens, which produces a visible color when aggregated at the corresponding ligand and can therefore be used in qualitative or semi-quantitative assays. With the development of colloidal gold research, in the surface of the gold nucleus can be certain modifications, mainly for the ligands containing sulfur, containing phosphide, phosphorus oxide, amino and carboxyl ligands. The method is less sensitive and cannot be used for quantitative testing to meet the requirements of clinical diagnosis.

### SUMMARY

In view of the above problem, the present disclosure is intended to provide a kit for testing a COVID-19 antigen and use thereof, which are applicable to clinical use and are capable of achieving quick, accurate, and low-cost testing.

Technical solutions of the present disclosure are as follows:
A kit for testing a COVID-19 antigen includes a test card, a conjugate pad and a reaction pad being arranged on the test card; wherein the conjugate pad is provided with a labeled antibody Ab1 and a labeled antibody Ab3 of the COVID-19 antigen, and a quality control line and a test line are arranged in parallel with an interval on the reaction pad, the test line being provided with a coating antibody Ab2, and the quality control line being provided with a coating antibody Ab4.

The present disclosure further provides a method for testing a COVID-19 antigen, wherein the test card in the kit as described above is used. The method includes:
sampling an oral pharyngeal mucosa liquid by inserting a disposable sampling swab from a human oral cavity into a throat and slowly rotating and wiping bilateral pharyngeal tonsils and a posterior wall of pharynx;
placing the sampling swab into an extraction bottle to which a sample extraction solution is added in advance, and sufficiently uniformly mixing the sample, such that the oral pharyngeal mucosa liquid remains in the extraction bottle; and
dropwise adding the sampled oral pharyngeal mucosa liquid into the test card in the kit for testing, and determining a test result according to the test line and the quality control line.

The present disclosure further provides a method for testing a COVID-19 antigen, wherein the test card in the kit as described above is used. The method includes:
sampling a nasal mucosa fluid by vertically inserting a disposable sampling swab into a nasal cavity to a depth of half of a length from an ear lobe to a nose tip;
placing the sampling swab into an extraction bottle to which a sample extraction solution is added in advance, and sufficiently uniformly mixing the sample, such that the nasal mucosa fluid remains in the extraction bottle; and
dropwise adding the sampled acquired nasal mucosa fluid into the test card in the kit for testing, and determining a test result according to the test line and the quality control line.

The present disclosure further provides a method for testing a COVID-19 antigen, wherein the test card in the kit as described above is used. The method includes:
sampling a nasal mucosa fluid or an oral pharyngeal mucosa fluid by using a disposable sampling swab;
placing the sampling swab into a single-lug tube to which a sample extraction solution is added in advance, and sufficiently uniformly mixing the sample, such that the nasal mucosa fluid or the oral pharyngeal mucosa fluid remains in the single-lug tube; and
dropwise adding the sampled nasal mucosa fluid or oral pharyngeal mucosa fluid into the test card in the kit for testing, and determining a test result according to the test line and the quality control line.

In the kit for testing the COVID-19 antigen according to the present disclosure, the conjugate pad in the test card in the kit is provided with the labeled antibody Ab1 and the labeled antibody Ab3 of the COVID-19 antigen, and the quality control line and the test line are arranged in parallel with the interval on the reaction pad, wherein the test line is provided with the coating antibody Ab2, and the quality control line is provided with the coating antibody Ab4. In this way, by the AIE immunofluorescence chromatographic technique, the AIE fluorescent microspheres are used as labels, and the test result of the test card is directly determined and read by using a fluorescent flashlight, or the test result is read by using a fluorescent tester. Such kits have the advantages of high sensitivity, strong specificity, simple operation, and the like.

### BRIEF DESCRIPTION OF THE DRAWINGS

FIG. 1 is a schematic structural view of a test card in a kit according to the present disclosure; and
FIG. 2 is a schematic structural view of a case fitted to the test card according to the present disclosure.

### DETAILED DESCRIPTION

Some exemplary embodiments of the present disclosure are described in detail hereinafter with reference to the accompanying drawings.

The AIE immunofluorescence chromatographic technique is a novel dry quantitative testing technique combining the AIE fluorescence technique and the traditional immunochromatographic technique. This technique not only has the advantages of simple operation, quick testing, and good portability of the colloidal gold immunochromatographic technique, but also achieves accuracy of a test result by the tracing enhancement technique of an aggregation-induced emission (AIE) material. Compared with the traditional fluorescence quick testing technique, this technical has merits of higher sensitivity, wider testing range, low cost, no background, and no quenching.

According to the present disclosure, in AIE immunofluorescence chromatographic technique, the content of the COVID-19 antigen in a nasal mucosa or oral pharyngeal mucosa fluid is tested by using the double antibody sandwich method. By means of process condition optimization, performance estimation, clinical application, and the like, an effective immunofluorescence chromatography-based kit for testing the COVID-19 antigen.

The present disclosure provides a kit for testing a COVID-19 antigen. The kit includes a test card, a disposable sampling swab, an extraction bottle, and a sample extraction solution disposed in a sample extraction solution bottle. A conjugate pad and a reaction pad are arranged on the test card; wherein the conjugate pad is provided with a labeled antibody Ab1 and a labeled antibody Ab3 of the COVID-19 antigen, and a quality control line and a test line are arranged in parallel with an interval on the reaction pad. The test line is provided with a coating antibody Ab2, and the quality control line is provided with a coating antibody Ab4.

The kit further includes a sample extraction solution (a phosphate buffer) for diluting a sample, an extraction bottle, and a disposable sampling swab. The disposable sampling swab is configured to sample an oral pharyngeal mucosa fluid or a nasal mucosa fluid of a human body. The sampled mucosa fluid is placed into the sample extraction solution in the extraction bottle, and the fluid sample to be tested is uniformly mixed. Afterwards, the sample to be tested is dropwisely added to the test card, and a test result is determined and read by the test line and the quality control line.

The sample extraction solution mainly includes 0.01 M BB, 1% PVP, 1% sodium chloride, and 1% Tween.

In the test card, a biological origin of the labeled antibody of the COVID-19 antigen in the conjugate pad is as illustrated by Ab1, and a biological origin of the coating antibody Ab2 of the COVID-19 antigen in the reaction pad is as illustrated by Ab2, a biological origin of the labeled antibody of the quality control line in the conjugate pad is as illustrated by Ab3, and a biological origin of the coating antibody of the quality control board in the reaction pad is as illustrated by Ab4. The details are as listed in Table 1.

**Table 1 Testing of antibodies of COVID-19 antigen, and biological origin of antigen**

| Main materials | Biological origin |
|---|---|
| Labeled antibody Ab1 of COVID-19 antigen | Murine monoclonal antibody IgG |
| Coating antibody Ab2 of COVID-19 antigen | Murine monoclonal antibody IgG |
| Chicken IgY labeled antibody Ab3 | Chicken antibody |
| Goat anti-chicken IgY coating antibody Ab4 | Goat antibody |

Further, the labeled antibody Ab1 contains AIE fluorescent microspheres (that is, an AIE fluorescently labeled antibody or an AIE fluorescent label), and the labeled antibody Ab3 contains AIE fluorescent microspheres (that is, an AIE fluorescently labeled antibody or an AIE fluorescently labeled antibody).

Preferably, a final concentration of the labeled antibody Ab1 0.3 mg/mL on the test card, a final concentration of the coating antibody Ab2 is 1 mg/mL on the test card, a final concentration of the labeled antibody Ab3 of the quality control line is 0.3 mg/mL on the test card, and a final concentration of the coating antibody Ab4 is 0.5 mg/mL on the test card.

During use of the test card, under chromatography of a capillary effect, the COVID-19 antigen in the sample is conjugated to the AIE fluorescently labeled antibody on the test card and is spread to the test region and captured by a COVID-19 monoclonal antibody coated by the test line, and hence an "antibody-antigen-fluorescent antibody" complex is formed. In the meantime, a concentration of the COVID-19 antigen is proportional to a fluorescence intensity of the complex, and negative and positive may be directly determined using a fluorescent flashlight.

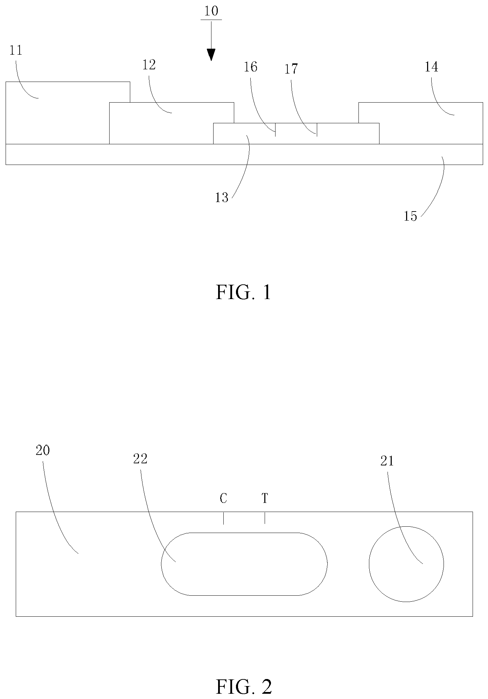

As illustrated in FIG. 1, a test card 10 further includes a sample pad 11, an absorbent pad 14, and a bottom plate 15. The bottom plate 15 is configured to an elongated strip. The test card 10 is formed by successively arranging, fixing and bonding the sample pad 11, a conjugate pad 12, a reaction pad 13, and the absorbent pad 14 onto a surface of the bottom plate 15. Two ends of the reaction pad 13 are respectively lapped with one end of the absorbent pad 14 and one end of the conjugate pad 12, the other end of the conjugate pad 12 is lapped with one end of the sample pad 11, the sample pad 11 and the conjugate pad 14 are respectively disposed on two ends of the bottom plate 15, and a quality control line (C line) 16 and a test line (T line) 17 are arranged on the reaction pad 13.

Further, as illustrated in FIG. 2, a case 20 is adaptively sleeved onto an outer surface of the test card 10; wherein the case 20 includes an observation window 22 and a well 21. The case 20 is made of a rigid material, for example, a rigid paper, a PVC, or the like. After the test card 10 is assembled into the case 20, the sample pad 11 is correspondingly arranged at the position of the well 21, and the reaction pad 12 is correspondingly arranged at the position of the observation window 22. Since the test card 10 is soft, the test operation is inconvenient. The case 20 is mainly to facilitate testing by using the test card 10, and strengthen rigidity of the test card 10, to ensure uprightness of the test card 10 during testing and prevent the test card 10 from being bent which affects the testing.

Further, during manufacturing, for protection of the test card 10 from moisture or contaminants, generally, the test card 10 assembled into the case 20 and a desiccant are packaged in a sealed fashion with an aluminum foil.

### In the test card 10:

The sample pad 11 is mainly composed of a glass cellulose membrane.

The conjugate pad 12 is mainly composed of a glass cellulose membrane, and the conjugate pad 12 is provided with a labeled antibody Ab1 with an AIE fluorescent label, and a labeled antibody Ab3 with an AIE fluorescent label. The labeled antibody Ab1 is a COVID-19 antibody Ab1, and the labeled antibody Ab3 is a chicken IgY antibody Ab3.

The reaction pad 13 is composed of a nitrocellulose (NC) membrane. The test line is coated with the antibody Ab2, that is, coated with the COVID-19 monoclonal antibody Ab2; and the quality control line is coated with the antibody Ab4, that is, coated by the goat anti-chicken IgY antibody.

The absorbent pad 14 is an H-1 absorbent paper.

The bottom plate 15 is a PVC sheet.

The present disclosure further provides a method for sampling a COVID-19 antigen. The method employs the kit as described above for testing the COVID-19 antigen. The method includes sampling using an oral pharyngeal swab and sampling using a nasal pharyngeal swab.

Oral pharyngeal swab: A sampling swab is totally inserted from a human oral cavity into a throat, and is slowly rotated to wipe bilateral pharyngeal tonsils while avoiding contact with the tongue.

Nasal pharyngeal swab: a distance from a nose tip to an ear lobe is measured and the distance is marked with the finger, and a sampling swab is inserted into a nasal cavity along a direction perpendicular the nose (face) to a depth of half of a length from the ear lobe to the nose tip. In the advent of a resistance, the swab reaches the nasal pharyngeal. In this case, the sampling swab is maintained at the nasal pharyngeal for 15 to 30s, and is slightly rotated for 3 to 5 times and then slowly taken out.

Sample storage: The collected sample shall be immediately subjected to testing. If the sample collected by the sampling swab is not immediately tested, the sample shall be immediately placed in a dry, disinfected and sealed plastic tube for storage.

Upon sample collection, the sample shall be immediately treated using the sample extraction solution provided by the kit. The treated sample may be preserved for 2h under room temperatures, 3h under temperatures of 2 to 8°C, and for 2 months under temperatures of -20±5°C. Note: The sample subjected to refrigeration preservation shall be restored to the room temperatures and then tested.

The sampling specifically includes the following steps:
opening a dropper bottle, and vertically dropwise adding 11 drops (about 400 µL) of the sample extraction solution into the extraction bottle;
sample collection: collecting the sample using an oral pharyngeal swab or a nasal pharyngeal swab;
upon sampling, placing the disposable sampling swab into the extraction bottle containing the sample extraction solution, soaking the swab in the sample extraction solution and extrusively rotating the swab for 10 times to sufficiently mix the sample, and then extruding the swab over the surface of the solution such that the fluid in the swab remains in the tube as much as possible;
connecting a disposable dropper to the extraction bottle containing the sample extraction solution;
opening an aluminum foil bag, taking out the test card, and placing the test card on a dry and clean platform;
vertically dropwise adding the solution in the extraction bottle into the well in the test card, wherein due to operation differences, if, 2 min after 3 drops of the sample extraction solution is added, it is found through the observation window that no solution layer is precipitated, 1 more drop of the sample extraction solution is added to the corresponding well under the observation window; and
within 10 to 15 min after sampling adding, determining and reading the test result on the test line and the quality control line by irradiating the observation window with the fluorescent flashlight, wherein the test result displayed after 30 min is of no clinical significance.

### Example 1 Preparation of kit

### 1. Preparation of the test card

### Step 1: Preparation of the conjugate pad

AIE fluorescence activation: 100 uL of AIE fluorescent microspheres were added to 800 uL of MES (2-morpholineethanesulfonic acid) in a centrifuge tube and sonicated for 3 min. 30 uL of EDC (1-ethyl-carbodiimide hydrochloride) and NHS (N-hydroxysuccinimide) (1: 1) activation solution was measured and mixed in the centrifuge tube, sonicated until it was evenly dispersed, equilibrated at room temperatures for a period of time, and centrifuged at 4°C for 15 min at 12000 r/min, and a supernatant was removed.

Antibody conjugation: 1 mL MES was added to the centrifuge tube, and sonicated for 3 min after vortexing, 0.3 mg of COVID-19 antibody was added and rotated for coupling reaction for 2 h, and after static placement for 5 min, 0.3 mg of chicken IgY antibody was added.

Closure and preservation: 100 uL of 10% BSA (bovine serum albumin) solution was added to the centrifuge tube and preserved for 30 min, and was centrifuged at 12000 r/min at 4°C for 15 min. The supernatant was removed, 1 mL of Tris buffer (Tris(hydroxymethyl)aminomethane) was added, sonicated for 3 min, and followed by centrifugation at 12000 r/min at 4°C for 15 min. The operation was repeated once. 1 mL of microsphere compound solution was added after the supernatant was moved, sonicated for 3 min, centrifuged at 2000 r/min for 3 min; and the precipitate was removed and kept at 4°C without irradiation from light.

Conjugate pad: A prepared fluorescent microparticle solution was uniformly sprayed on a glass fiber of 1.5-1.8 cm at a rate of 6 uL/cm, and dried at 55 °C for 3 to 4 h.

### Step 2: Preparation and treatment of the sample pad

Preparation of a treatment solution: 0.1 M BB, 1% sucrose, 1% trehalose, and 1% PVP K30 (polyvinylpyrrolidone).

Treatment of the sample pad: the treatment solution is evenly applied to a 0.8-1.0 cm sample pad by an amount of 3-4 mL/piece (width: 17-18, length: 30 cm), and dried at 55°C for 6-8 h.

### Step 3: Preparation of the reaction pad

Preparation of a coating solution: 0.05 M Tris (Tris(hydroxymethyl)aminomethane), and 1% sucrose. Scoring:
1) Test line: A reaction pad (that is, a nitrocellulose membrane, NC membrane) was pasted on the bottom plate (a PVC sheet ), a COVID-19 coating antibody Ab2 was prepared with the coating solution at a concentration of 1 mg/mL, uniformly sprayed on the nitrocellulose membrane at 1.2 µL/cm using a quantitative film-spraying instrument, and dried in an air-blowing drying oven at 50°C for 24 hours to prepare a reaction pad containing the test line;
2) Quality control line: A coating antibody Ab4 of goat anti-chicken IgY with a concentration of 0.5 mg/mL was uniformly sprayed on the reaction pad containing the test line using a quantitative film sprayer at a concentration of 1.0 µL/cm, and dried in a blast drying oven at 50°C for 24 h to prepare a reaction pad containing the test line and the quality control line.

Step 4: Assembly of the test card: The treated sample pad, conjugate pad and absorbent pad were successively attached to the PVC sheet, and the assembled PVC sheet is cut into an immunochromatographic test card with a width of 3.85 mm by a strip cutter.

### 2. Sample testing and result determination

Preparation of a sample extraction solution: 0.01 M BB, 1% PVP (polyvinylpyrrolidone), 1% sodium chloride, and 1% Tween.

### Sample testing:

opening a dropper bottle, and vertically dropwise adding 11 drops (about 400 µL) of the sample extraction solution into the extraction bottle;
sample collection: collecting the sample using an oral pharyngeal swab or a nasal pharyngeal swab;
upon sampling, placing the disposable sampling swab into the extraction bottle containing the sample extraction solution, soaking the swab in the sample extraction solution and extrusively rotating the swab for 10 times to sufficiently mix the sample, and then extruding the swab over the surface of the solution such that the fluid in the swab remains in the tube as much as possible;
connecting a disposable dropper to the extraction bottle containing the sample extraction solution;
opening an aluminum foil bag, taking out the test card, and placing the test card on a dry and clean platform; and
vertically dropwise adding the solution in the extraction bottle into the well in the test card, wherein due to operation differences, if, 2 min after 3 drops of the sample extraction solution is added, it is found through the observation window that no solution layer is precipitated, 1 more drop of the sample extraction solution is added to the corresponding well under the observation window; and determining and reading the test result 15 min later with the fluorescent flashlight. In the fluorescence test, no matter whether it is negative or positive, the quality control line shall show a fluorescence band, and in this case, it is proved that the test result is valid.

### 3. Packaging of the kit

The composition, packaging and quantity (25 pieces/kit) of the CVOID-19 antigen kit in human oral pharyngeal or nasal pharyngeal samples are listed in Table 2.

**Table 2 Composition, packaging and quantity of the kit**

| Serial number | Component | Main component | Specification (µL) | Quantity |
|---|---|---|---|---|
| 1 | Test card for the COVID-19 antigen | This product is composed of a card case and a test strip, which is composed of a sample pad, a conjugate pad (containing fluorescently labeled COVID-19 antibody and fluorescently labeled chicken IgY labeled antibody), a reaction pad, i.e., a nitrocellulose membrane (test line encapsulated with a COVID-19 antigen monoclonal antibody and a quality control line sheep anti-chicken IgY-coated antibody), an absorbent pad, and a PVC backing plate. | For one person (one piece)/bag | 25 |
| 2 | Sample extraction solution | Phosphate buffer | Bottle | 25 |
| 3 | Extraction bottle | / | piece | 25 |
| 4 | Disposable sampling swab | / | piece | 25 |
| 5 | SPECIFICATION | / | Parts | 1 |

In addition, the present disclosure provides another embodiment:

The extraction bottle in the kit for detecting the CVOID-19 antigen according to the present disclosure can be replaced with a single-lug tube.

The components, packaging and quantity of the kit (25 pieces/kit) are listed in Table 3.

**Table 3 Composition, packaging and quantity of the kit**

| Serial number | Component | Main component | Specification (µL) | Quantity |
|---|---|---|---|---|
| 1 | Test card for the COVID-19 antigen | This product is composed of a card case and a test strip. The test strip is composed of a sample pad, a conjugate pad (containing a fluorescently labeled COVID-19 antibody and a fluorescently labeled chicken IgY labeled antibody), a reaction pad, i.e., a nitrocellulose membrane (a test line coated with a COVID-19 monoclonal antibody and a quality control line coated with a goat anti-chicken IgY coated antibody), an absorbent pad, and a PVC bottom plate. | For one person (one piece)/bag | 25 |
| 2 | Sample extraction solution | Phosphate buffer | Bottle | 25 |
| 3 | Disposable sampling swab | / | piece | 25 |
| 4 | SPECIFICATION | / | Parts | 1 |

A method for testing a COVID-19 antigen using the kit as described above includes the following steps:
sampling a nasal mucosa fluid or an oral pharyngeal mucosa fluid by using a disposable sampling swab;
placing the sampling swab into a single-lug tube to which a sample extraction solution is added in advance, and sufficiently uniformly mixing the sample, such that the nasal mucosa fluid or the oral pharyngeal mucosa fluid remains in the single-lug tube; and
dropwise adding the sampled nasal mucosa fluid or oral pharyngeal mucosa fluid into the test card in the kit for testing, and determining a test result according to the test line and the quality control line.

The sampling specifically includes the following steps:
1. sample collection: collecting the sample using an oral pharyngeal swab or a nasal pharyngeal swab;
2. upon sampling, placing the disposable sampling swab into a single-lug tube containing the sample extraction solution, soaking the swab in the sample extraction solution and extrusively rotating the swab for 10 times to sufficiently mix the sample, and then extruding the swab over the surface of the solution such that the fluid in the swab remains in the tube as much as possible;
3. connecting a disposable dropper to the single-lug tube containing the sample extraction solution;
4. opening an aluminum foil bag, taking out the test card, and placing the test card on a dry and clean platform; and
5. vertically dropwise adding the solution in the single-lug tube into the well in the test card, wherein due to operation differences, if, 2 min after 4 drops of the sample extraction solution is added, it is found through the observation window that no solution layer is precipitated, 1 more drop of the sample extraction solution is added to the corresponding well under the observation window; and
6. within 10 to 15 min after sampling adding, determining and reading the test result on the test line and the quality control line by irradiating the observation window with the fluorescent flashlight, wherein the test result displayed after 30 min is of no clinical significance. In the fluorescence test, no matter whether it is negative or positive, the quality control line shall show a fluorescence band, and in this case, it is proved that the test result is valid.

It should be understood that described above are merely exemplary embodiments of the present disclosure, which are not intended to limit the projection scope of the present disclosure. The projection scope of the present disclosure shall be subject to the appended claims.

## Claims

1. A kit for testing a coronavirus disease 2019 (COVID-19) antigen, comprising a test card, a conjugate pad and a reaction pad being arranged on the test card; wherein the conjugate pad is provided with a labeled antibody Ab1 and a labeled antibody Ab3 of the COVID-19 antigen, and a quality control line and a test line are arranged in parallel with an interval on the reaction pad, the test line being provided with a coating antibody Ab2, and the quality control line being provided with a coating antibody Ab4.

2. The kit according to claim 1, wherein the labeled antibody Ab1 is biologically derived from a murine monoclonal antibody IgG.

3. The kit according to claim 1, wherein the coating antibody Ab2 is biologically derived from a murine monoclonal antibody IgG.

4. The kit according to claim 1, wherein the labeled antibody Ab3 is biologically derived from a chicken antibody.

5. The kit according to claim 1, wherein the coating antibody Ab4 is biologically derived from a goat antibody.

6. The kit according to claim 1, wherein the labeled antibody Ab1 and the labeled antibody Ab3 both comprise AIE fluorescent microspheres.

7. The kit according to claim 1, wherein a final concentration of the labeled antibody Ab1 is 0.3 mg/ml, and a final concentration of the coating antibody Ab2 is 1 mg/mL.

8. The kit according to claim 1, wherein a final concentration of the labeled antibody Ab3 is 0.3 mg/ml, and a final concentration of the coating antibody Ab4 is 0.5 mg/mL.

9. The kit according to claim 1, further comprising a sample extraction solution for extracting a sample, an extraction bottle, and a disposable sampling swab.

10. The kit according to claim 9, wherein the sample extraction solution comprises 0.01 M BB, 1% PVP, 1% sodium chloride, and 1% Tween.

11. The kit according to claim 1, wherein the labeled antibody Ab1 comprises AIE fluorescent microspheres, and the labeled antibody Ab3 comprises AIE fluorescent microspheres.

12. The kit according to claim 1, wherein a final concentration of the labeled antibody Ab1 0.3 mg/mL on the test card, a final concentration of the coating antibody Ab2 is 1 mg/mL on the test card, a final concentration of the labeled antibody Ab3 of the quality control line is 0.3 mg/mL on the test card, and a final concentration of the coating antibody Ab4 is 0.5 mg/mL on the test card.

13. The kit according to claim 1, wherein the test card further comprises a sample pad, an absorbent pad, and a bottom plate; wherein the test card is formed by successively arranging, fixing and bonding the sample pad, the conjugate pad, the reaction pad, and the absorbent pad onto a surface of the bottom plate, wherein two ends of the reaction pad are respectively lapped with one end of the absorbent pad and one end of the conjugate pad, the other end of the conjugate pad is lapped with one end of the sample pad, the sample pad and the conjugate pad are respectively disposed on two ends of the bottom plate, and the quality control line and the test line are arranged on the reaction pad.

14. The kit according to claim 13, wherein the sample pad is composed of a glass cellulose membrane.

15. The kit according to claim 13, wherein the absorbent pad is an H-1 absorbent paper.

16. The kit according to claim 13, wherein the bottom plate is a PVC sheet.

17. The kit according to claim 1, wherein a case is adaptively sleeved onto an outer surface of the test card; wherein the case comprises an observation window and a well.

18. A method for testing a coronavirus disease 2019 (COVID-19) antigen using the kit as defined in any one of claims 1 to 17, comprising:
sampling an oral pharyngeal mucosa liquid by inserting a disposable sampling swab from a human oral cavity into a throat and slowly rotating and wiping bilateral pharyngeal tonsils and a posterior wall of pharynx;
placing the sampling swab into an extraction bottle to which a sample extraction solution is added in advance, and sufficiently uniformly mixing the sample, such that the oral pharyngeal mucosa liquid remains in the extraction bottle; and
dropwise adding the sampled oral pharyngeal mucosa liquid into the test card in the kit for testing, and determining a test result according to the test line and the quality control line.

19. A method for testing a coronavirus disease 2019 (COVID-19) antigen using the kit as defined in any one of claims 1 to 17, comprising:
sampling a nasal mucosa fluid by vertically inserting a disposable sampling swab into a nasal cavity to a depth of half of a length from an ear lobe to a nose tip;
placing the sampling swab into an extraction bottle to which a sample extraction solution is added in advance, and sufficiently uniformly mixing the sample, such that the nasal mucosa fluid remains in the extraction bottle; and
dropwise adding the sampled acquired nasal mucosa fluid into the test card in the kit for testing, and determining a test result according to the test line and the quality control line.

20. A method for testing a coronavirus disease 2019 (COVID-19) antigen using the kit as defined in any one of claims 1 to 17, comprising:
sampling a nasal mucosa fluid or an oral pharyngeal mucosa fluid by using a disposable sampling swab;
placing the sampling swab into a single-lug tube to which a sample extraction solution is added in advance, and sufficiently uniformly mixing the sample, such that the nasal mucosa fluid or the oral pharyngeal mucosa fluid remains in the single-lug tube; and
dropwise adding the sampled nasal mucosa fluid or oral pharyngeal mucosa fluid into the test card in the kit for testing, and determining a test result according to the test line and the quality control line.
